(19) Europäisches Patentamt
European Patent Office
Office européen des brevets

(11) EP 4 692 081 A1

(12) **EUROPEAN PATENT APPLICATION**
published in accordance with Art. 153(4) EPC

(43) Date of publication:
**11.02.2026 Bulletin 2026/07**

(21) Application number: **24777935.8**

(22) Date of filing: **22.03.2024**

(51) International Patent Classification (IPC):
***C07D 413/14*** $^{(2006.01)}$ ***C07D 498/00*** $^{(2006.01)}$

(52) Cooperative Patent Classification (CPC):
**C07D 413/14; C07D 498/00**

(86) International application number:
**PCT/CN2024/083383**

(87) International publication number:
**WO 2024/199146 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.03.2023 CN 202310294912**

(71) Applicant: **Zhangzhou Pien Tze Huang Pharmaceutical Co., Ltd.**
**Fujian Province, 363000 (CN)**

(72) Inventors:
- **LIN, Jinxia**
  **Zhangzhou, Fujian 363000 (CN)**
- **SHEN, Guoying**
  **Zhangzhou, Fujian 363000 (CN)**
- **YANG, Limei**
  **Zhangzhou, Fujian 363000 (CN)**
- **WU, Huakai**
  **Zhangzhou, Fujian 363000 (CN)**
- **ZHANG, Xiaoqin**
  **Zhangzhou, Fujian 363000 (CN)**
- **WANG, Shaohui**
  **Shanghai 200131 (CN)**
- **XING, Shaoting**
  **Shanghai 200131 (CN)**
- **XU, Yu**
  **Shanghai 200131 (CN)**

(74) Representative: **Dehns**
**10 Old Bailey**
**London EC4M 7NG (GB)**

# (54) PREPARATION METHOD FOR NAPHTHOISOXAZOLE COMPOUND

(57) Disclosed is a preparation method for a naphthoisoxazole compound. Specifically, disclosed is a preparation method for a compound of formula (I) and an intermediate thereof.

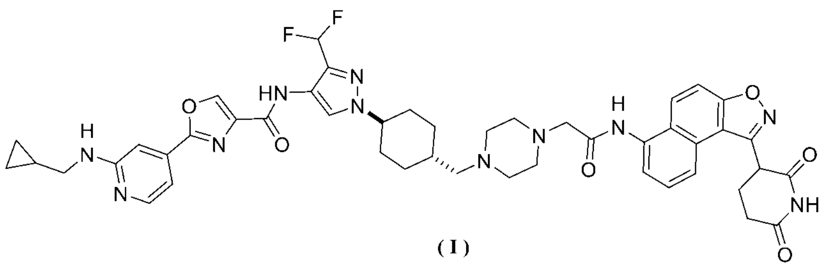

(I)

EP 4 692 081 A1

## Description

The present application claims the following priority:

The present application claims the priority and right of the Chinese patent application No. 2023102949123 filed with the China National Intellectual Property Administration on March 24, 2023. The contents of the Chinese patent application are incorporated herein by reference in its entirety.

TECHNICAL FIELD

The present disclosure relates to a method for preparing a naphthoisoxazole compound, specifically to a method for preparing a compound of formula (I) and an intermediate thereof.

BACKGROUND

Interleukin-1 receptor-associated kinase 4 (IRAK4) is a serine/threonine kinase that plays an important role in the signal transduction of Toll/IL-1 receptors (TIRs). Multiple IRAK enzymes are key components in the signal transduction pathways mediated by interleukin-1 receptors (IL-1Rs) and Toll-like receptors (TLRs). There are four members in the mammalian IRAK family: IRAK-1, IRAK-2, IRAK-3, and IRAK-4. These proteins are characterized by a typical N-terminal death domain that mediates interactions with MyD88 family adapter proteins and a central kinase domain. When MyD88 and IRAK4 are recruited to the Tollinterleukin domain, activation leads to phosphorylation of IRAK-1 and/or IRAK-2, thereby inducing TRAF6 binding. Subsequent signal transduction leads to the activation of NF-κB and the release of various cytokines and chemokines. The MyD88 L265P mutation, occurring in 91% of Waldenström macroglobulinemia, 29% of ABC-DLBCL, 9% of MALT lymphoma, and 3% of CLL, is associated with constitutively active signalosomes, wherein IRAK-4-mediated phosphorylation of IRAK-1 promotes the assembly of other signaling proteins that drive the survival of these cancers.

IRAK4 possesses kinase-independent scaffolding functions. Similar scaffolding functions have been shown in human fibroblasts, where kinase-dead IRAK4 can restore IL-1-induced NF-κB signaling to levels comparable to WT IRAK4. IRAK4 may be targeted for degradation, thereby offering therapeutic opportunities for treating autoimmune diseases, inflammatory, and tumor diseases. The specific degradation of IRAK4 can be achieved by using heterobifunctional small molecules to recruit IRAK4 to ubiquitin ligases, thereby promoting ubiquitination and proteasomal degradation of IRAK4. For example, thalidomide derivatives, such as lenalidomide or pomalidomide, have been reported to recruit potential protein substrates to Cereblon (CRBN), a component of the ubiquitin ligase complex. Cereblon is a protein encoded by the CRBN gene in humans. CRBN orthologs are highly conserved from plants to humans, highlighting their physiological importance. Cereblon forms an E3 ubiquitin ligase complex (CUL4A) with damaged DNA-binding protein 1 (DDB1), Cullin-4A, and the regulator of cullins-1 (ROC 1). CRBN, as an important target for antitumor and immunomodulatory drugs, has been demonstrated to exhibit definitive therapeutic efficacy in various hematologic malignancies, skin diseases such as erythema nodosum leprosum, and autoimmune diseases such as systemic lupus erythematosus.

Proteolysis targeting chimera (PROTAC) is one of the new hotspots in the field of drug research and development in recent years. It utilizes small-molecule compounds to recruit a specific ubiquitin ligase and achieves protein degradation through ubiquitination of the target protein. PROTAC is a class of dual-target chimera molecules composed of three parts: a ligand targeting the protein of interest, a ligand recruiting E3 ubiquitin ligase, and a linker connecting the two. Compared to traditional small-molecule inhibitors, PROTACs exhibit potential advantages in drug dosage, selectivity, resistance, and modulation of "undruggable targets", and have now transitioned from basic research to clinical trials.

To further enhance drug accessibility and facilitate scale-up production, it is necessary to develop new processes for obtaining this drug.

SUMMARY

The present disclosure provides a method for preparing compound 6 using compound 4 and compound 5, wherein the reaction step is as follows:

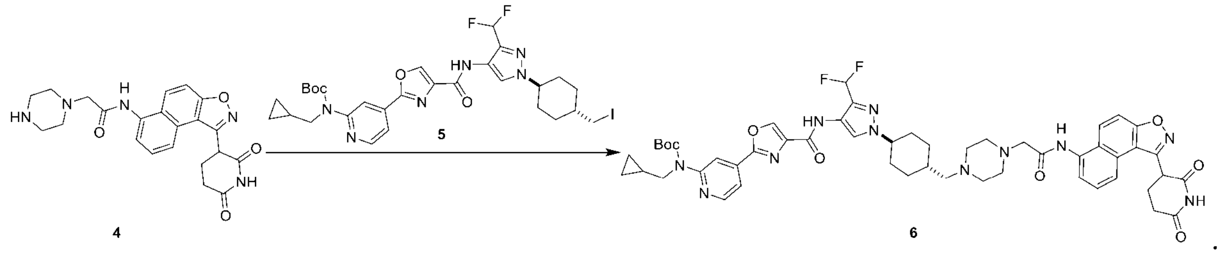

4 5 6

The present disclosure also provides a method for preparing a compound of formula (I) using compound 1, wherein the reaction steps are as follows:

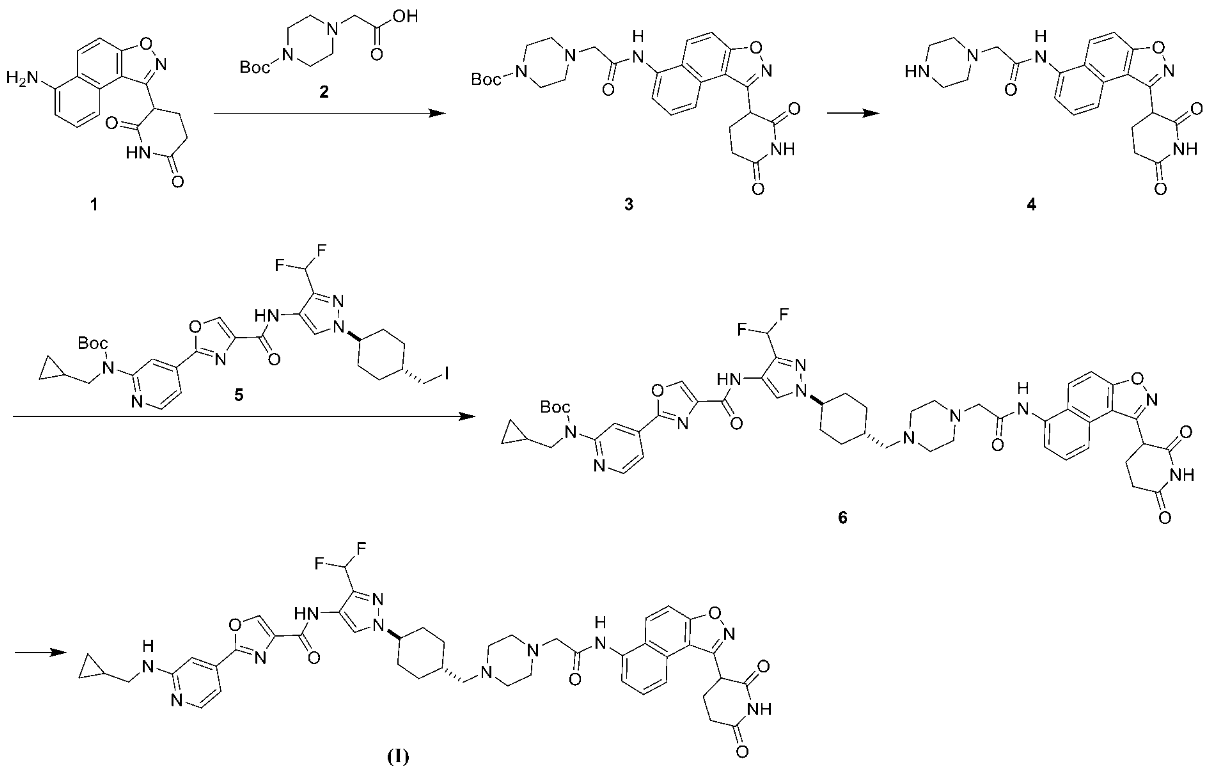

1 2 3 4 5 6 (I)

.

In some embodiments of the present disclosure, in the method for preparing compound 6 using compound 4 and compound 5,

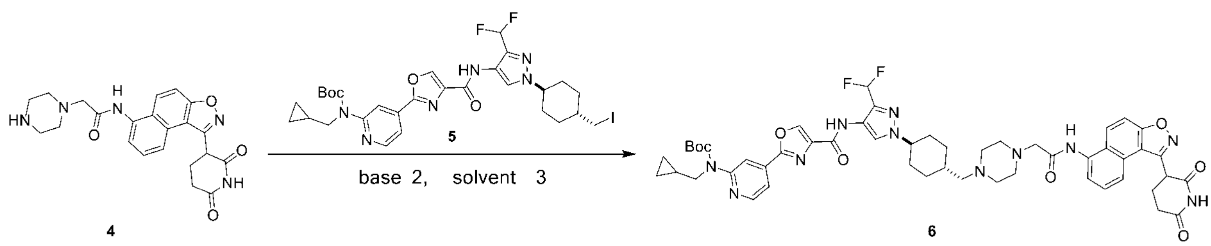


4 5 6

the equivalent ratio of compound 4 to compound 5 is 0.8:1 to 1:1;
base 2 is selected from the group consisting of TEA and DIEA;
solvent 3 is acetonitrile.

In some embodiments of the present disclosure, the reaction temperature is 70 to 80°C, and other variables are as defined in the present disclosure.

In some embodiments of the present disclosure, the equivalent ratio of compound 4 to compound 5 is 0.85:1 to

0.95:1, and other variables are as defined in the present disclosure.

In some embodiments of the present disclosure, the equivalent ratio of compound 4 to compound 5 is 0.88:1, and other variables are as defined in the present disclosure.

In some embodiments of the present disclosure, in the method for preparing the compound of formula (I) using compound 1,

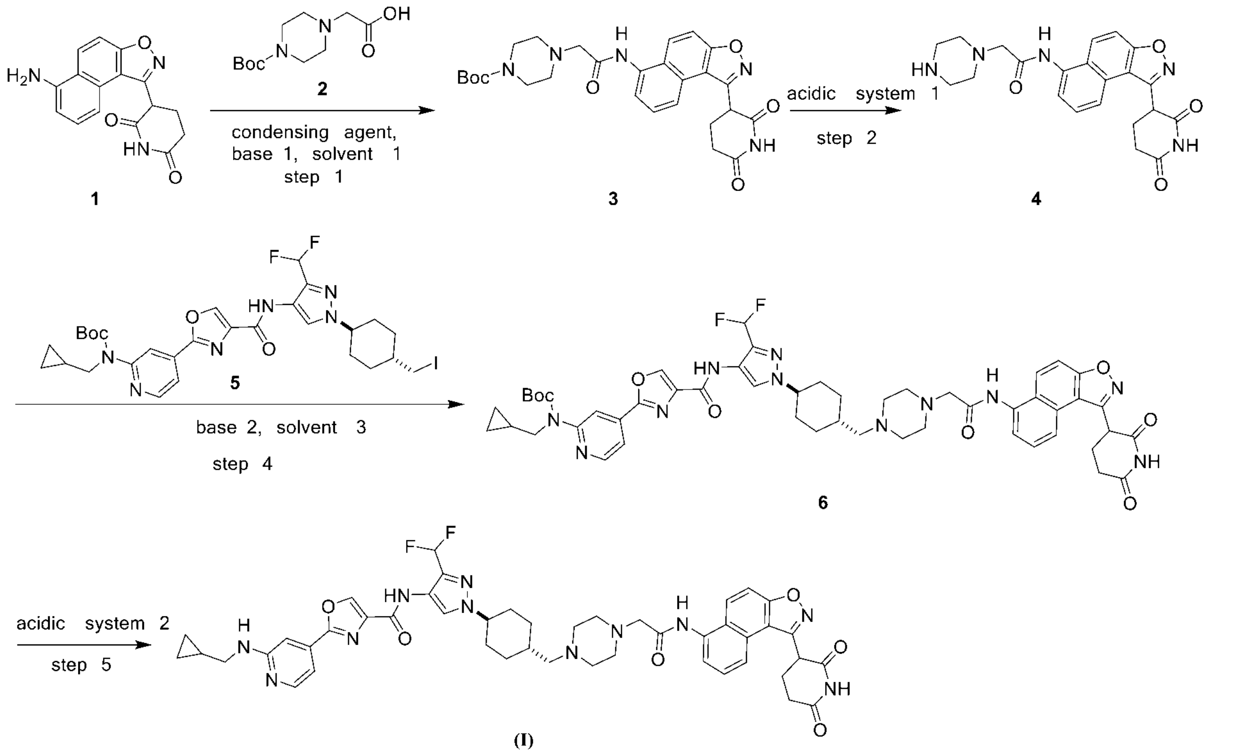


1 3 4 6 (I)

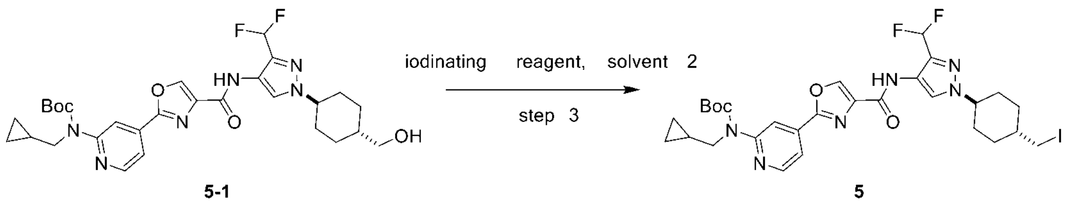


5-1 5

,

the condensing agent is selected from the group consisting of HATU, benzenesulfonyl chloride, and p-toluenesulfonyl chloride;
base 1 is selected from the group consisting of TEA and DIEA;
solvent 1 is selected from the group consisting of DMF, DMSO, and NMP;
acidic system 1 is selected from the group consisting of a solution of hydrogen chloride in ethyl acetate and TFA;
acidic system 2 is selected from the group consisting of a solution of hydrogen chloride in ethyl acetate and TFA;
base 2 is selected from the group consisting of TEA, DIEA, and sodium bicarbonate;
solvent 3 is acetonitrile;
the iodinating reagent is $PPh_3/Im/I_2$;
solvent 2 is DCM.

In some embodiments of the present disclosure, the condensing agent is HATU; acidic system 1 is TFA; acidic system 2 is a solution of hydrogen chloride in ethyl acetate, wherein the concentration of the solution of hydrogen chloride in ethyl acetate is 4 mol/L; base 1 is DIEA; base 2 is DIEA.

In some embodiments of the present disclosure, the reaction temperature of step 5 is 40 to 50°C, and other variables are as defined in the present disclosure.

In some embodiments of the present disclosure, the reaction time of step 5 is 0.5 to 3 hours, and other variables

are as defined in the present disclosure.

**Technical Effect**

The process for synthesizing the compound of formula (I) provided by the present disclosure offers a novel method for preparing a naphthoisoxazole compound, with the beneficial effects as follows: The entire process has mild reaction conditions, simple and convenient operation, and a simple and effective purification method, and is more convenient for industrial production.

Specifically:

1) In the synthetic process for preparing the compound of formula (I) according to the present disclosure, the purification methods mainly include slurrying or crystallization, as well as extraction operations, which are simple and easy to perform, avoiding column chromatography, thereby being more convenient for industrial production.
2) The process in step 3 can avoid configuration inversion and the generation of isomers, making the purification process simpler.
3) No highly toxic or flammable/explosive reagents are used throughout the preparation process, ensuring the safety of the process production.

**Definition and Description:**

Unless otherwise specified, the following terms and phrases, when used herein, have the following meanings. A specific phrase or term should not be considered indefinite or unclear in the absence of a particular definition, but should be understood in the ordinary sense. When a trading name appears herein, it is intended to refer to its corresponding commodity or active ingredient thereof.

The intermediate compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include, but are not limited to, the examples of the present disclosure. Those skilled in the art may appropriately modify aspects such as raw materials and process conditions based on the content of the present disclosure to achieve corresponding other objectives. Such related modifications do not depart from the content of the present disclosure. All similar substitutions and alterations are obvious to those skilled in the art and are deemed to be included within the scope of the present disclosure.

The chemical reactions of the specific embodiments of the present disclosure are completed in a suitable solvent, and the solvent must be appropriate for the chemical changes of the present disclosure and the required reagents and materials thereof. In order to obtain the compounds of the present disclosure, it is sometimes necessary for those skilled in the art to modify or select the synthetic steps or reaction processes on the basis of the existing embodiments.

An important consideration in any synthetic route planning by those skilled in the art is the selection of appropriate protecting groups for reactive functional groups (such as the amino group in the present disclosure).

All solvents used in the present disclosure are commercially available.

The following abbreviations are used in the present disclosure: Boc represents *tert*-butoxycarbonyl; EtOAc represents ethyl acetate; HATU represents 2-(7-azabenzotriazol-1-yl)-*N*,*N*,*N'*,*N'*-tetramethyluronium hexafluorophosphate; ACN represents acetonitrile; DIEA represents N,N-diisopropylethylamine; Im represents imidazole; DCM represents dichloromethane; DMF represents *N,N*-dimethylformamide; $PPh_3$ represents triphenylphosphine.

Compounds are named by conventional nomenclature methods in the art or using ChemDraw® software, and commercially available compounds adopt the catalog names provided by suppliers.

DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific examples have also been disclosed; for those skilled in the art, it is obvious to make various modifications and improvements to the specific examples of the present disclosure without departing from the spirit and scope of the present disclosure.

Example 1: Preparation of the compound of formula (I)

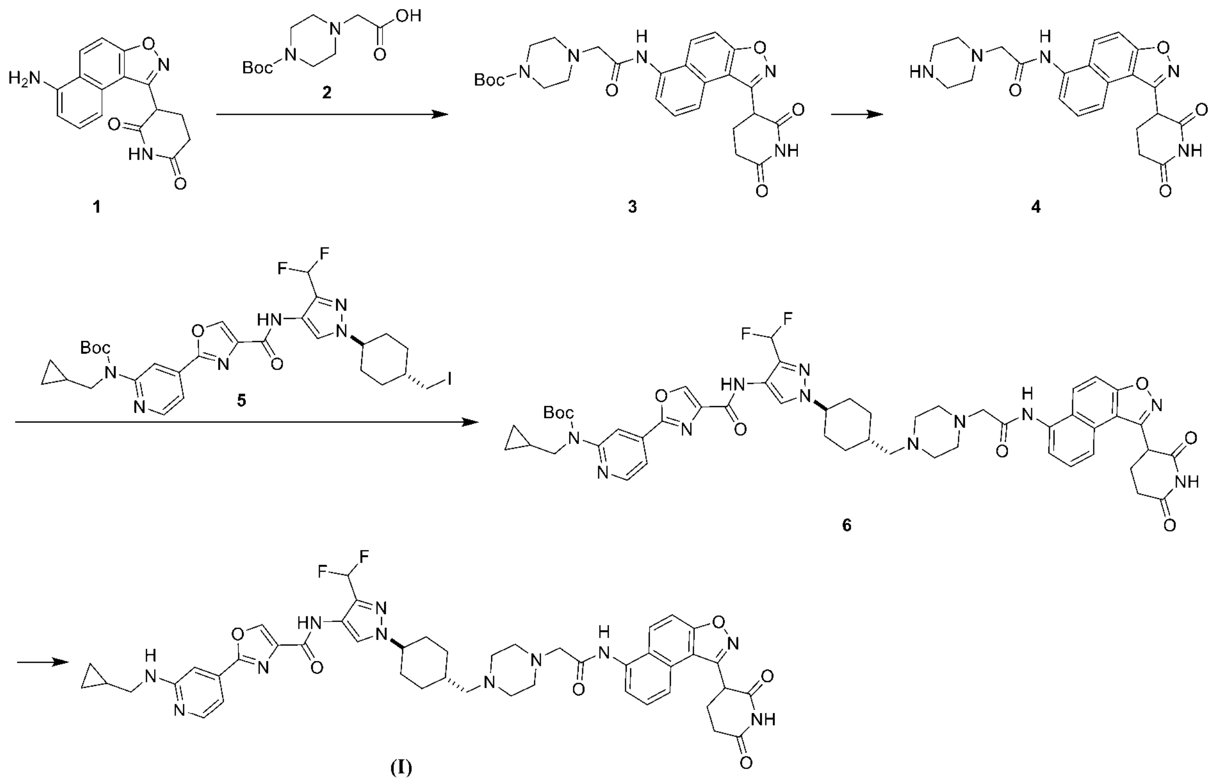

1 2 3 4 5 6 (I)

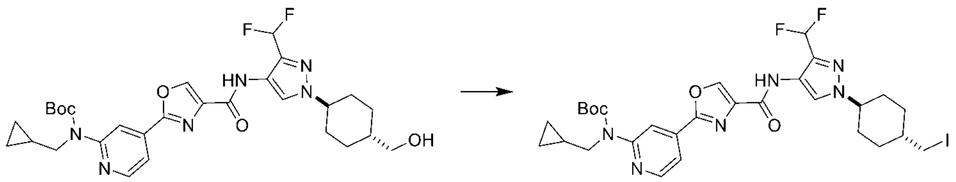

5-1 5

Step 1: Synthesis of compound **3**

Under a slight nitrogen flow at 10 to 15°C, *N,N*-dimethylformamide (4.8 L) was added to a reaction vessel, followed by the addition of compound **2** (702.94 g), the hydrochloride of compound **1** (800 g), and *N,N*-diisopropylethylamine (739.62 g), with the temperature maintained at 10 to 20°C throughout the process. Finally, 2-(7-azabenzotriazol-1-yl)-*N,N,N',N'*-tetramethyluronium hexafluorophosphate (1.31 kg) was added in 10 batches. The system was exothermic during the addition, and the temperature was maintained at 15 to 30°C throughout the process. After the addition was completed, the mixture was stirred for 4 to 24 hours while maintaining the temperature at 20 to 30°C. After the reaction was completed, acetic acid (137.46 g) was added to the reaction vessel while maintaining the temperature at 20 to 30°C, and the mixture was stirred for 20 to 40 minutes. Methanol (110.02 g) was then added, and the mixture was stirred for 1.5 to 3 hours while maintaining the temperature. Methyl *tert*-butyl ether (12 L) was transferred into the reaction vessel using a peristaltic pump, and the mixture was stirred for 9 to 12 hours while maintaining the temperature at 10 to 20°C. The mixture was filtered, and the filter cake was rinsed with methyl *tert-butyl* ether (2 L × 2). The filter cake was collected.

Under a nitrogen atmosphere at 10 to 20°C, water (4.8 L) and isopropanol (480 mL) were transferred into a 10 L reaction flask. The filter cake was added, and the mixture was stirred for 1.5 to 2.5 hours. The mixture was filtered, and the filter cake was rinsed with a mixed solvent of water and isopropanol (volume ratio: water/isopropanol = 10/1, 500 mL × 2). The filter cake was collected and dried to obtain compound **3** (953.2 g, purity: 98.7%, yield: 75.8%). $^1$H NMR (400MHz, DMSO_$d_6$) δ: 11.14 (s, 1H), 10.13 (s, 1H), 8.19 (d, *J*=9.6 Hz, 1H), 8.11 (d, *J*=7.2 Hz, 1H), 8.00 (d, *J*=9.2 Hz, 1H), 7.82-7.65 (m, 2H), 5.09 (dd, *J*=4.8, 11.2 Hz, 1H), 3.53-3.40 (m, 4H), 3.32 (s, 2H), 2.87-2.80 (m, 1H), 2.70-2.66 (m, 1H), 2.65-2.55 (m,

5H), 2.44-2.34 (m, 1H), 2.44-2.34 (m, 1H).

Step 2: Synthesis of the trifluoroacetate salt of compound **4**

Under a slight nitrogen flow at 15 to 25°C, dichloromethane (9.3 L) was transferred into a reaction vessel, followed by the addition of compound **3** (930 g) and trifluoroacetic acid (2.14 kg). After the addition was completed, the mixture was stirred for 0.5 to 1 hour while maintaining the temperature. The reaction mixture was gradually warmed to 35 to 42°C and stirred for 3 to 5 hours. After the reaction was completed, the reaction mixture was cooled to 15 to 25°C, stirred for 1 to 3 hours while maintaining the temperature, and then filtered. The filter cake was rinsed with dichloromethane (930 mL × 3), and the filter cake was collected.

Under a slight nitrogen flow at 15 to 25°C, water (7.4 L) and isopropanol (1.5 L) were transferred into a reaction vessel, followed by the addition of imidazole (971.1 g). After the mixture was stirred until clear, the filter cake was added, and the mixture was stirred for 0.5 to 1 hour while maintaining the temperature. The reaction mixture was cooled to 0 to 10°C and stirred for 10 to 16 hours while maintaining the temperature. The mixture was filtered, and the filter cake was rinsed with water (500 mL × 2). The filter cake was collected and transferred into a 10 L reaction flask, followed by the addition of water (4.18 L). The mixture was stirred at 5 to 15°C for 0.5 to 1 hour. The mixture was filtered, and the filter cake was sequentially rinsed with water (500 mL × 3) and isopropanol (500 mL × 2). The filter cake was collected and dried to obtain the trifluoroacetate salt of compound **4** (722 g, purity: 99.9%, yield: 77.9%). $^1H$ NMR (400MHz, DMSO_$d_6$) δ: 11.15 (s, 1H), 10.15 (s, 1H), 8.73 (s, 2H), 8.21 (d, *J*=9.6 Hz, 1H), 8.14 (d, *J*=7.6 Hz, 1H), 7.97 (d, *J*=9.2 Hz, 1H), 7.79-7.61 (m, 2H), 5.09 (dd, *J*=4.8, 11.6 Hz, 1H), 3.42 (s, 2H), 3.28-3.16 (m, 4H), 2.94-2.78 (m, 5H), 2.72-2.54 (m, 2H), 2.45-2.34 (m, 1H).

Step 3: Synthesis of compound **5**

Under a slight nitrogen flow at 10 to 15°C, dichloromethane (25 L) was added to a reaction vessel, followed by the addition of compound **5-1** (2.5 kg, 4.26 mol), triphenylphosphine (1.34 kg, 5.11 mol), and imidazole (435.18 g, 6.39 mol). The reaction mixture was cooled to 0 to 5°C, and elemental iodine (1.41 kg, 5.54 mol) was added in 6 batches, with the temperature maintained at 0 to 5°C during the addition. After the addition was completed, the mixture was warmed to 15 to 25°C and stirred for 16 hours. After the reaction was completed, a saturated sodium sulfite solution (3.7 L) and water (12.5 L) were added to the reaction mixture, followed by stirring for 30 minutes. The mixture was allowed to stand for phase separation, and the aqueous phase was extracted again with dichloromethane (5 L × 2). The organic phases were combined. The organic phase was sequentially washed with saturated brine (12.5 L) and water (12.5 L), dried over anhydrous sodium sulfate, filtered, and the filtrates from two batches were combined and concentrated under reduced pressure to obtain a crude product.

Ethanol (25 L) was added to the crude product, and the mixture was heated to 70°C until clear, then stirred for 1 hour while maintaining the temperature, slowly cooled to room temperature, and stirred for an additional 16 hours. The two batches were combined and filtered. The filter cake was rinsed with ethanol (5 L × 2), and the filter cake was collected. Methanol (25 L) was added to the filter cake, and the mixture was stirred at room temperature for 1 hour. After filtration, the filter cake was rinsed with methanol (2.5 L × 2, 2 L × 2, 1.5 L × 2), collected, and dried to obtain compound **5** (4.8 kg, purity: 99.0%, yield: 80.8%). $^1H$ NMR (400MHz, DMSO_$d_6$) δ: 9.77 (s, 1H), 9.01 (s, 1H), 8.59 (d, *J*=5.6 Hz, 1H), 8.29 (s, 1H), 8.19 (s, 1H), 7.68 (dd, *J*=1.6, 9.2 Hz, 1H), 7.15 (t, *J*=54.4 Hz, 1H), 4.21 (tt, *J*=4.0, 12.0 Hz, 1H), 3.86 (d, *J*=6.8 Hz, 1H), 3.17 (d, *J*=6.0 Hz, 2H), 2.10-1.91 (m, 4H), 1.88-1.74 (m, 2H), 1.51 (s, 9H), 1.49-1.43 (m, 1H), 1.26-1.12 (m, 3H), 0.44-0.37 (m, 2H), 0.27-0.20 (m, 2H).

Step 4: Synthesis of compound **6**

Under a slight nitrogen flow at 15 to 25°C, acetonitrile (8 L), the trifluoroacetate salt of compound **4** (679.63 g), and compound **5** (1.005 kg) were added to a reaction vessel, followed by the addition of *N,N*-diisopropylethylamine (356.26 g). After the addition was completed, the reaction mixture was heated to 75 to 80°C and reacted for 22 to 24 hours while maintaining the temperature. After the reaction was completed, the mixture was slowly cooled to 15 to 25°C (cooling rate of 10 to 20°C/hour when the temperature was >50°C; cooling rate of 5 to 10°C/hour when the temperature was ≤50°C) and stirred for 1 to 2 hours while maintaining the temperature, during which solids gradually precipitated. The stirring rate was controlled at 100 to 150 rpm. The mixture was filtered, and the filter cake was rinsed with acetonitrile (600 mL × 2) and collected.

Under a slight nitrogen flow at 15 to 25°C, acetonitrile (8 L) was added to a reaction vessel, followed by the addition of the filter cake. The reaction mixture was heated to 70 to 80°C, and after all solids were dissolved, stirring was continued for 0.5 to 1 hour. The stirring rate was controlled at 100 to 150 rpm, and a gradient cooling to 15 to 25°C was initiated (cooling rate of 10 to 20°C/hour when the temperature was >65°C; cooling rate of 5 to 10°C/hour when the temperature was ≤65°C). The mixture was stirred for 1 to 2 hours while maintaining the temperature. The mixture was

filtered, and the filter cake was rinsed with acetonitrile (600 mL × 2), collected, and dried to obtain compound **6** (1.03 kg, purity: 98.5%, yield: 79.5%). $^1$H NMR (400MHz, DMSO_$d_6$) δ: 11.17 (s, 1H), 10.13 (s, 1H), 9.79 (s, 1H), 9.01 (s, 1H), 8.59 (d, *J*=4.8 Hz, 1H), 8.30 (s, 1H), 8.21-8.15 (m, 2H), 8.14-8.06 (m, 1H), 8.01 (d, *J*=9.2 Hz, 1H), 7.79 (d, *J*=7.6 Hz, 1H), 7.73 (d, *J*=8.0 Hz, 1H), 7.70-7.66 (m, 1H), 7.16 (t, *J*=54.4 Hz, 1H), 5.09 (dd, *J*=4.4, 11.2 Hz, 1H), 4.28-4.15 (m, 1H), 3.86 (d, *J*=6.8 Hz, 1H), 3.35 (s, 4H), 3.29 (s, 2H), 2.91-2.80 (m, 1H), 2.74-2.57 (m, 6H), 2.43-2.36 (m, 1H), 2.22-2.14 (m, 2H), 2.10-2.01 (m, 3H), 1.97-1.87 (m, 2H), 1.84-1.70 (m, 2H), 1.68-1.57 (m, 1H), 1.51 (s, 9H), 1.24-0.98 (m, 3H), 0.44-0.37 (m, 2H), 0.27-0.20 (m, 2H).

Step 5: Synthesis of the compound of formula **(I)**

Under a slight nitrogen flow at 15 to 25°C, a solution of hydrogen chloride in ethyl acetate (4 M, 10 L) was transferred into a reaction vessel. Compound **6** (1.01 kg) was added in batches. After the addition was completed, the mixture was stirred for 20 to 40 minutes while maintaining the temperature. The reaction mixture was then gradually heated to 40 to 50°C and stirred for 1 to 3 hours while maintaining the temperature. After the reaction was completed, the reaction mixture was cooled to 20 to 25°C and filtered. The filter cake was rinsed with ethyl acetate (1 L), collected, and purged with nitrogen for 18 to 24 hours.

Under a slight nitrogen flow at 15 to 25°C, dimethyl sulfoxide (3.5 L) was transferred into a reactor. The dried filter cake was added and stirred for 0.5 hours. After complete dissolution (heating to 40 to 50°C may assist dissolution), the mixture was filtered, and the filter cake was rinsed with dimethyl sulfoxide (120 mL). The filtrate was collected.

Under a slight nitrogen flow at 15 to 25°C, water (40 L) was transferred into a reaction vessel. Imidazole (661.73 g) was added and stirred for 10 minutes to dissolve. The filtrate was then added over approximately 0.5 hours, followed by stirring for 2 hours while maintaining the temperature. The mixture was filtered, and the filter cake was rinsed with water (1 L) and collected. Water (10 L) was transferred into a reaction vessel, and the filter cake was added. The mixture was stirred for 0.5 hours. The mixture was filtered, and the filter cake was sequentially washed with water (1 L × 2) and ethanol (1 L × 2). The filter cake was collected and dried to obtain the compound of formula (I) (873.9 g, purity: 98.4%, yield: 95.8%). $^1$H NMR (400MHz, DMSO_$d_6$) δ: 11.15 (s, 1H), 10.12 (s, 1H), 9.70 (s, 1H), 8.93 (s, 1H), 8.19 (t, *J*=4.4 Hz, 2H), 8.15 (d, *J*=5.6 Hz, 1H), 8.13-8.07 (m, 1H), 8.01 (d, *J*=9.2 Hz, 1H), 7.80 (d, *J*=7.2 Hz, 1H), 7.72 (t, *J*=8.0 Hz, 1H), 7.31-7.03 (m, 3H), 7.01 (dd, *J*=1.2, 5.2 Hz, 1H), 5.09 (dd, *J*=4.8, 11.6 Hz, 1H), 4.27-4.15 (m, 1H), 3.37-3.23 (m, 4H), 3.18 (t, *J*=6.4 Hz, 2H), 2.91-2.80 (m, 1H), 2.73-2.53 (m, 7H), 2.44-2.33 (m, 2H), 2.23-2.14 (m, 2H), 2.10-2.02 (m, 2H), 1.97-1.87 (m, 2H), 1.84-1.70 (m, 2H), 1.68-1.55 (m, 1H), 1.14-0.98 (m, 3H), 0.49-0.41 (m, 2H), 0.25-0.18 (m, 2H).

**Bioassay**

**Experimental Example 1: Evaluation of target protein degradation effect in K562 IRAK4-HiBiT cells**

**Objective:** In this experiment, the degradation effect of the test compound on the target protein IRAK4 in K562 IRAK4-HiBiT cells was detected.

**Experimental materials:**

1. Cells and culture medium

Cells: K562 IRAK4-HiBiT cells

Culture medium: RPMI 1640 + 10% fetal bovine serum + 2 mM glutamine + 1 mM sodium pyruvate + penicillin/streptomycin

Positive control: 1000 nM; negative control: 0.1% DMSO

Table 1. Reagents and consumables

| Reagents and Consumables | Manufacturer | Cat. No. |
|---|---|---|
| RPMI 1640 | Gibco | Cat# 31800 |
| Fetal Bovine Serum (FBS) | Biosera | #FB-1058/500 |
| Penicillin/Streptomycin | Biosera | #LM-A4118 |
| DPBS | Invitrogen | #14190 |

(continued)

| Reagents and Consumables | Manufacturer | Cat. No. |
|---|---|---|
| 0.25% Trypsin/EDTA Solution | Invitrogen | #25200 |
| Glutamine | Invitrogen | Cat# 35050061 |
| Sodium Pyruvate | Invitrogen | Cat# 11360070 |
| Nano-Glo® HiBiT Assay Kit | Promega | Promega# N3040 |
| 384-Well White Plate, Flat Bottom | Corning | Cat# 3570 |
| 384_LDV Compound Plate | Labcyte | Cat# LP-0200 |

Table 2. Instruments

| Instrument Name | Manufacturer | Instrument Model |
|---|---|---|
| ECHO Pipette | Labcyte | Echo 550 |
| Bravo Automated Liquid Handling Platform | Agilent | 16050-101 |
| Envision Plate Reader | PerkinElmer | 2104 |
| Autosampler | Thermo Fisher | Multidrop Combi |
| Cell Counter | Thermo | Countess II FL |

**Experimental scheme:**

**Day 1**

1. Compound preparation

(1) The test compound in powder form was dissolved in DMSO to 10 mM as a storage concentration, and 9 μL of the 10 mM test compound was manually pipetted to column 1 and column 13 of an LDV plate using a pipette.
(2) 6 μL of DMSO was added to columns 2 to 12 and 14 to 24 using Multidrop Combi.
(3) The test compound was 3-fold diluted (3 μL + 6 μL) from columns 1 to 11 and 13 to 23 using Bravo.
(4) 25 nL of compound solution (columns 1 to 24 of the LDV plate) was transferred to an assay plate using Echo according to the plate layout.
(5) 25 nL of 1 mM positive control solution was transferred to the assay plate as a 100% degradation control *(i.e.,* LC, HPE), and 25 nL of DMSO was transferred to the assay plate as a 0% control *(i.e.,* HC, ZPE) using Echo.

2. Cell seeding

(1) The cell culture medium was discarded, and the cells were washed once with DPBS, digested with trypsin, and counted to prepare a cell suspension of $2 \times 10^{-5}$ cells/mL.
(2) 25 μL/well of cell suspension was added to the assay plate containing the test compound using Multidrop Combi at medium speed.
(3) The assay plate containing cells was returned to an incubator with 5% $CO_2$ at 37°C and incubated for 16 to 18 hours.

**Day 2**

(1) 25 μL/well of assay reagent (NanoGlo lysis buffer + substrate + LgBit protein) was added to the assay plate using Multidrop Combi at high speed, and the plate was shaken for 10 minutes.
(2) The plate was centrifuged at 2000 rpm for 1 minute to remove air bubbles.
(3) The plate was read on Envision using US Luminescence detection method.

3. Data analysis

The degradation rate (DR) of the test compound was calculated using the following formula: DR (%) = (RLU vehicle control - RLU compound) / (RLU vehicle control - RLU positive control) * 100%, where the vehicle control served as the blank control. The degradation rates of compounds at different concentrations were calculated in Excel, and XLFit software was used to generate inhibition curves and calculate relevant parameters, including minimum degradation rate, maximum degradation rate, and $DC_{50}$.
The test results are shown in Table **3.**

Table **3.** Target protein degradation effect of the compound of the present disclosure in K562 IRAK4-HiBiT cells

| Compound No. | $DC_{50}$ (nM) | Maximum Degradation Rate (%) |
|---|---|---|
| Hydrochloride of Compound of Formula (I) | 4.87 | 108.46 |

**Conclusion:** The compound of the present disclosure exhibits an excellent target protein degradation effect in K562 IRAK4-HiBiT cells.

**Experimental Example 2: In cell western analysis of IKZF1 and IKZF3 protein expression levels in MM.1S Cells**

**Objective:** The experiment was conducted to evaluate the degradation effects of test compounds on IKZF1 and IKZF3 proteins in MM.1S cells by detecting their impact on the expression levels of IKZF1 and IKZF3 proteins in MM.1S cells.

**Experimental materials:**

Cell line: MM.1S cell (obtained from ATCC; Cat. No. CRL-2974)
Negative control: 0.1% DMSO

Table 4. Reagents and consumables

| Reagents and Consumables | Supplier | Cat. No. |
|---|---|---|
| 96-Well Black Clear-Bottom Plate for ELISA | JingAn Biological | J09603 |
| D-PBS Buffer | Bioleaper | P220610030051 |
| Tissue Fixative Solution | Meilumbio | MA0192-Jan-28G |
| TritonX-100 Solution (10% Sterile) | Beyotime | ST797-500ML |
| Intercept Blocking Buffer | LI-COR | 211114 |
| Ikaros (D6N9Y) Rabbit mAb | Cell Signaling | 14859S |
| Aiolos (D1C1E) Rabbit mAb | Cell Signaling | 15103S |
| GAPDH Mouse mAb | Proteintech | 60004-1-Ig |
| Tween 20 | Sinopharm Chemical Reagent Co., Ltd. | 30189328 |
| IRDye 800CW Goat AntiMouse | LI-COR | D00812-08 |
| IRDye 680RD Goat Anti-Rabbit | LI-COR | D00891-05 |

Table 5. Instruments

| Instrument Name | Supplier | Instrument Model |
|---|---|---|
| Dual-Color Infrared Laser Imager | LI-COR | Odyssey-CLx |
| Decolorization Shaker | QILNBEIER | TS-1000 |
| Refrigerator | Haier Group | BCD-256KT |

**Experimental scheme:**

1) MM.1S cells in the logarithmic growth phase were seeded into a 96-well plate at $1.2 \times 10^5$ cells per well and cultured overnight.
2) On the second day, drug treatment was initiated with a starting concentration of 300 nM, followed by a 3-fold dilution in triplicate wells, with 10 concentration gradients (containing DMSO), and incubated for 24 hours in a culture incubator.
3) The mixture was centrifuged, and the cell supernatant was carefully removed. Then, 150 μL of 4% paraformaldehyde fixative solution was added along the well wall without touching the bottom cells, followed by incubation at room temperature for 20 min.
4) Permeabilization solution was prepared by adding 0.5 mL of 10% Triton X-100 to 49.5 mL of PBS and mixing thoroughly.
5) 200 μL of the permeabilization solution was added along the well wall without touching the bottom cells, and incubated at room temperature on a shaker for 5 min.
6) The washing step was repeated 4 times.
7) 150 μL of Licor INERCEPT blocking buffer (intertent Blacking buffer) was added along the well wall without touching the bottom cells, and incubated at room temperature on a shaker for 1.5 hours.
8) Ikaros (D6N9Y) Rabbit mAb, Aiolos (DIC1E) Rabbit mAb, and GAPDH mouse mAb (proteintech, 60004-1-Ig) were diluted at a ratio of 1:100 using antibody diluent.
9) 50 μL of the mixed antibodies was added to each well in triplicate, and incubated overnight at 4°C on a shaker.
10) PBST (PBS containing 0.1% Tween 20) was prepared.
11) The primary antibody was removed, and 200 μL of PBST was added along the well wall without touching the bottom cells. The mixture was incubated at room temperature on a shaker for 5 min.
12) The washing step was repeated 4 times.
13) The secondary antibody diluent was prepared by adding Tween 20 to Licor INTERCEPT blocking buffer at a final concentration of 0.2%.
14) The fluorescent secondary antibody was diluted in the dark (1:800 dilution). To 400 μL of the secondary antibody diluent, 0.5 μL each of IRDye 800CW and IRDye 680CW was added (the corresponding fluorescent secondary antibody for the primary antibody).
15) 50 μL of the diluted fluorescent secondary antibody was added to each well, followed by incubation at room temperature on a shaker in the dark for 60 min.
16) The secondary antibody was removed, and 200 μL of PBST was added along the well wall without touching the bottom cells. The plate was then incubated at room temperature on a shaker in the dark for 5 min.
17) The washing step was repeated 4 times. Immediately after washing, the plate was scanned using the Odyssey Gel Imaging System at dual wavelengths of 700 nm and 800 nm.

3. Data analysis

GraphPad Prism 6 software was utilized to fit the curve with the inhibition rate data, and the $DC_{50}$ value was calculated.

$$\text{Protein inhibition rate} = (1 - \text{RLs} / \text{RLv}) * 100\%$$

$$\text{RR (Raw Ratio)} = 700\ \text{nm} / 800\ \text{nm}$$

$$\text{RLs} = \text{RR of sample-treated cells}$$

$$\text{RLv} = \text{RR of vehicle-treated cells}$$

The test results are shown in Table **6.**

Table **6.** Degradation effects of the compound of the present disclosure on IKZF1 and IKZF3 proteins in MM.1S cells

| Compound No. | Protein | $DC_{50}$ (nM) | Maximum Degradation Rate Dmax (%) |
|---|---|---|---|
| Hydrochloride of Compound of Formula (I) | IKZF 1 | 23.40 | 72.32 |
| | IKZF3 | 20.64 | 73.46 |

**Conclusion:**

The compound of the present disclosure exhibits excellent target protein degradation effects on IKZF1 and IKZF3 proteins in MM.1S cells.

**Experimental Example 3: Evaluation of anti-proliferative effect in lymphoma cell lines OCI-LY10 and TMD-8**

**Objective:** This experiment was conducted to detect the inhibitory effects of the test compound on cell proliferation in diffuse large B-cell lymphoma cell lines **OCI-LY10 and TMD-8.**

**Experimental materials:**

Table 7. Cell lines and culture methods

| Cell line | Tumor Type | Growth Characteristics | Culture Medium |
|---|---|---|---|
| OCI-LY10 | Lymphoma | Suspension | IMDM + 20% FBS + 55 μM β-Mercaptoethanol |
| TMD-8 | Lymphoma | Suspension | MEM + 10% FBS |

Table 8. Culture media and reagents

| Culture Media and Reagents | Manufacturer | Cat. No. |
|---|---|---|
| Dulbecco's PBS | Hyclone | SH30256.01 |
| Fetal Bovine Serum (FBS) | GIBCO | 10099-141 |
| Antibiotic-Antimycotic | GIBCO | 15240-062 |
| 0.25% Trypsin | GIBCO | 25200072 |
| DMSO | SIGMA | D2650 |
| β-Mercaptoethanol | SIGMA | 60-24-2 |
| MEM | GIBCO | 11095-080 |
| IMDM | GIBCO | 12440-053 |

1. Multi-Well Plate

Greiner CELLSTAR® 96-well plate, flat-bottom white (with lid and clear bottom), # 3610.

2. Reagents and instruments for cell viability assay

(1) Promega CellTiter-Glo luminescent cell viability assay kit (Promega-G7573).
(2) 2104 EnVision® plate reader, PerkinElmer.

**Experimental scheme:**

1. Cell culture

The tumor cell lines were cultured under the above culture conditions in an incubator at 37°C and 5% $CO_2$. The cells were passaged periodically, and those in the logarithmic growth phase were harvested for seeding.

2. Cell seeding

(1) The cells were stained with trypan blue, and the viable cells were counted.
(2) The cell concentration was adjusted to an appropriate concentration.

Table 9. Cell lines and seeding densities

| **Cell Line** | **Seeding Density (cells/well)** |
|---|---|
| OCI-LY10 | 5000 |
| TMD-8 | 5000 |

(3) 100 μL of cell suspension was added to each well of the culture plate as shown in the table above.
(4) The culture plate was incubated overnight in an incubator at 37°C, 5% $CO_2$, and 100% relative humidity.
3. Preparation of compound storage plate
Preparation of stock solution storage plate with 1000 times the initial concentration of the compound: The compound was serially diluted with DMSO from the highest concentration to the lowest concentration. The solution was prepared as needed each time.
4. Preparation of 1000× compound working solution and compound treatment of cells

(1) Preparation of 5× working solution from the initial compound concentration: When performing a 3-fold dilution of the compound, 30 μL of the compound was pipetted from the 1000× stock solution. Subsequently, 20 μL of DMSO was added to the subsequent wells, and 10 μL was sequentially pipetted from the previous concentration to the next. When performing a 5-fold dilution of the compound, 30 μL of the compound was pipetted from the 1000× stock solution, followed by the addition of 24 μL of DMSO to the subsequent wells. Then, 6 μL was sequentially pipetted from the previous concentration to the next. In the vehicle control, 20 μL of DMSO was added. The 1000× compound was diluted 200-fold with culture medium, specifically by adding 1 μL of the 1000× diluted compound to 199 μL of culture medium, followed by pipetting up and down to mix thoroughly.
(2) Drug addition: 25 μL of the 5× compound was added to the cell culture plate.
(3) The 96-well cell plate was returned to the incubator for culturing OCI-LY10 (3-fold or 5-fold dilution, incubated with the drug for 5 days) and TMD-8 (3-fold or 5-fold dilution, incubated with the drug for 5 days).

5. CellTiter-Glo luminescent cell viability assay
The following steps were conducted in accordance with the instructions of the Promega CellTiter-Glo luminescent cell viability assay kit (Promega-G7573).

(1) CellTiter-Glo buffer was thawed and brought to room temperature.
(2) CellTiter-Glo substrate was brought to room temperature.
(3) CellTiter-Glo working solution was prepared by adding 10 mL of CellTiter-Glo buffer to a vial of CellTiter-Glo substrate to dissolve the substrate.
(4) The mixture was slowly vortexed and shaken to be fully dissolved.
(5) The cell culture plate was taken out and left for 30 minutes to equilibrate to room temperature.
(6) 60 μL of CellTiter-Glo working solution was added per well (equal to half the volume of cell culture medium per well). The cell plate was wrapped in aluminum foil to be protected from light.
(7) The culture plate was shaken on an orbital shaker for 2 minutes to induce cell lysis.
(8) The culture plate was left at room temperature for 10 minutes to stabilize the luminescent signal.
(9) The luminescent signal was detected on a 2104 EnVision plate reader.

6. Data analysis
The inhibition rate (IR) of the test compound was calculated using the following formula: IR (%) = (1 - RLU compound / RLU vehicle control) * 100%. The inhibition rates of compounds at different concentrations were calculated in Excel, and then GraphPad Prism software was used to plot inhibition curves and calculate relevant parameters, including minimum inhibition rate, maximum inhibition rate, and $IC_{50}$.
The test results are shown in Table **10.**

Table **10.** Inhibitory effects of the compound of the present disclosure on cell proliferation in cell lines OCI-LY10 and TMD-8

| Compound No. | OCI-LY10 | | TMD-8 | |
|---|---|---|---|---|
| | $IC_{50}$ (nM) | Maximum Inhibition Rate (%) | $IC_{50}$ (nM) | Maximum Inhibition Rate (%) |
| Hydrochloride of Compound of Formula (I) | 13.66 | 94.54 | 10.34 | 89.86 |
| "/" indicates not detected. | | | | |

**Conclusion:** The compound of the present disclosure exhibits excellent inhibitory effects on cell proliferation in both lymphoma cell lines OCI-LY10 and TMD-8.

**Experimental Example 4: Evaluation of anti-proliferative effect in lymphoma cell line SU-DHL-2**

**Objective:** This experiment was conducted to detect the inhibitory effect of the test compound on cell proliferation in the lymphoma cell line **SU-DHL-2.**

Table **11.** Cell line and culture method

| Cell Line | Tumor Type | Growth Characteristic | Culture Method |
|---|---|---|---|
| SU-DHL-2 | Lympho ma | Suspension | RPMI1640 + 10% FBS (EXCELL) + 1% Penicillin/Streptomycin |

Table **12.** Culture media and reagents

| Culture Media and Reagents | Manufacturer | Cat. No. |
|---|---|---|
| RPMI 1640 | GIBCO | 22400-089 |
| Dulbecco's PBS | Thermo | SH30028.02B |
| Fetal Bovine Serum (FBS) | Hyclone | 11H233 |
| Antibiotic-Antimycotic | GIBCO | 15240-062 |
| DMSO | SIGMA | D2650 |

1. Multi-well plate
Greiner CELLSTAR 384-well plate, flat-bottom black (with lid), # 781090.
2. Reagents and instruments for cell viability assay

(1) Promega CellTiter-Glo luminescent cell viability assay kit (Promega-G7573).
(2) 2104 EnVision® plate reader, PerkinElmer.

**Experimental scheme:**
1. Cell culture
The tumor cell lines were cultured under the above culture conditions in an incubator at 37°C and 5% $CO_2$. The cells were passaged periodically, and those in the logarithmic growth phase were harvested for seeding.
2. Cell seeding
(1) The cells were stained with trypan blue, and the viable cells were counted.
(2) The cell concentration was adjusted to an appropriate concentration.

Table **13.** Cell line and seeding density

| **Cell Line** | **Seeding Density (cells/well)** |
|---|---|
| SU-DHL-2 | 1500 |

(3) 50 $\mu$L of cell suspension was added to each well of the culture plate as shown in the table above.
(4) The culture plate was incubated overnight in an incubator at 37°C, 5% $CO_2$, and 100% relative humidity.
3. Preparation of compound storage plate
The drug was administered with an Echo655 instrument. A dosing volume of 50 nL was used, with a final DMSO concentration of 0.1%. The culture plate was centrifuged at 1000 rpm for 1 min and incubated at 37°C, 5% $CO_2$, and 100% relative humidity for 4 days.
4. CellTiter-Glo luminescent cell viability assay
The following steps were conducted in accordance with the instructions of the Promega CellTiter-Glo luminescent cell viability assay kit (Promega-G7573).

(1) CellTiter-Glo buffer was thawed and brought to room temperature.
(2) CellTiter-Glo substrate was brought to room temperature.
(3) CellTiter-Glo working solution was prepared by adding CellTiter-Glo buffer to a vial of CellTiter-Glo substrate to dissolve the substrate.
(4) The mixture was slowly vortexed and shaken to be fully dissolved.
(5) The cell culture plate was taken out and left for 30 minutes to equilibrate to room temperature.

(6) 25 μL (equal to half the volume of cell culture medium in each well) of CellTiter-Glo working solution was added to each well. The cell plate was wrapped in aluminum foil to be protected from light.
(7) The culture plate was shaken on an orbital shaker for 2 minutes to induce cell lysis.
(8) The culture plate was left at room temperature for 10 minutes to stabilize the luminescent signal.
(9) The luminescent signal was detected using a 2104 EnVision plate reader.

5. Data analysis
The inhibition rate (IR) of the test compounds was calculated using the following formula: IR (%) = (1 - (RLU compound - RLU blank control) / (RLU vehicle control - RLU blank control)) * 100%. The inhibition rates of compounds at different concentrations were calculated in Excel, and then GraphPad Prism software was used to generate inhibition curves and calculate relevant parameters, including the minimum inhibition rate, maximum inhibition rate, and $IC_{50}$.

Table **14.** Inhibitory effects of the compound of the present disclosure on cell proliferation in cell line SU-DHL-2

| Compound No. | SU-DHL-2 $IC_{50}$ (nM) | Maximum Inhibition Rate (%) |
|---|---|---|
| Hydrochloride of Compound of Formula (I) | 28.70 | 93.55 |

**Conclusion:**

The compound of the present disclosure exhibits an excellent inhibitory effect on cell proliferation in the lymphoma cell line SU-DHL-2.

**Experimental Example 5: pharmacokinetic evaluation of the compound in mice**

**Objective:**

C57BL/6 or C57 male mice were selected as the test animals for this study. The LC/MS/MS method was employed to quantitatively determine the plasma drug concentrations at various time points after intravenous injection or intragastric administration of the test compound, aiming to evaluate the pharmacokinetic characteristics of the compound of the present disclosure in mice.

**Experimental materials:**

C57BL/6 or C57 mice (male, 20 to 30g, 7 to 10 weeks old, Beijing Vital River).
A clear solution of the test compound was injected intravenously into the tail vein of C57BL/6 mice (fasted overnight) (vehicle: 10% DMSO/10% solutol/80% $H_2O$), or administered intragastrically to C57 mice (fed). For intravenous injection, 50 μL of blood was collected via the cheek puncture at 0 h (pre-administration) and 0.083 h, 0.25 h, 0.5 h, 1 h, 2 h, 4 h, 6 h, 8 h, and 24 h after administration, and placed in an anticoagulant tube added with heparin sodium. The mixture was thoroughly vortexed and mixed, and centrifuged at 6000 g for 3 minutes at 2 to 8°C. For oral administration, blood was collected via the cheek puncture at 0 h (pre-administration) and 0.083 h, 0.25 h, 0.5 h, 1 h, 2 h, 4 h, 6 h, 8 h, and 24 h after administration, and placed in an anticoagulant tube added with heparin sodium. The mixture was thoroughly vortexed and mixed, and centrifuged at 6000 g for 3 minutes at 2 to 8°C. Plasma drug concentrations were determined by the LC-MS/MS method, and relevant pharmacokinetic parameters were calculated using Phoenix WinNonlin 8.2.0 pharmacokinetic software with non-compartmental model linear-log trapezoidal method.
The test results are shown in Table **15.**

Table **15.** Pharmacokinetic parameters of the compound of the present disclosure in mice

| Compound No. | Pharmacokinetic Parameters in Mice | | | | | | | |
|---|---|---|---|---|---|---|---|---|
| | Intravenous Injection (2 mg/kg) (fasted) | | | | Intragastric administration (10 mg/kg) (fed) | | | |
| | Plasma Clearance (mL/min /kg) | Elimination Half-Life (h) | Apparent Volume of Distribution (L/kg) | Area Under the Drug-Time Curve (0-inf, μM·h) | Peak Concentration (μM) | Time to Peak Concentration (h) | Area Under the Drug-Time Curve (0-inf, μM·h) | Bioavai -lability F (%) |
| Hydrochloride of Compound of Formula (I) | 7.0 | 4.22 | 1.6 | 5.40 | 0.54 | 1.00 | 4.47 | 18.90 |
| "/" indicates not detected. | | | | | | | | |

**Conclusion:** The compound of the present disclosure has high oral systemic plasma exposure ($AUC_{0-inf}$). It has superior pharmacokinetic properties in rodents such as mice.

**Experimental Example 7: Pharmacokinetic evaluation of the compound in beagle dogs**

**Objective:**

Male beagle dogs were selected as the experimental animals in this study. The LC/MS/MS method was employed to quantitatively determine the plasma drug concentrations at different time points after intravenous injection or intragastric administration of the test compound, aiming to evaluate the pharmacokinetic characteristics of the compound of the present disclosure in beagle dogs.

**Experimental materials:**

Beagle dogs (male, 7 to 10 kg, Beijing Mars Biotechnology Co., Ltd.).

**Experimental operation:**

A clear solution of the test compound was slowly injected intravenously into beagle dogs (fed) via the peripheral vein (vehicle: 5% DMSO/10% Solutol/85% $H_2O$), or administered intragastrically to beagle dogs (fed). For intravenous administration, 0.5 mL of blood was collected from the peripheral vein at 0.083, 0.25, 0.5, 1, 2, 4, 6, 8, and 24 h after administration, placed in EDTA-2K anticoagulant tubes, and centrifuged at 3200 g for 10 minutes at 2 to 8°C to separate the supernatant. For intragastric administration, 0.5 mL of blood was collected from the peripheral vein at 0.083, 0.25, 0.5, 1, 2, 4, 6, 8, and 24 h after administration, placed in EDTA-2K anticoagulant tubes, and centrifuged at 3200 g for 10 minutes at 2 to 8°C to separate the supernatant. Plasma drug concentrations were determined by the LC-MS/MS method, and relevant pharmacokinetic parameters were calculated using Phoenix WinNonlin 6.3 pharmacokinetic software with non-compartmental model linear-log trapezoidal method.
The test results are shown in Table **16.**

Table **16.** Pharmacokinetic parameters of the compound of the present disclosure in Beagle dogs

| Compound No. | Pharmacokinetic Parameters in Beagle Dogs | | | | | | | |
|---|---|---|---|---|---|---|---|---|
| | Intravenous Injection (0.5 mg/kg) | | | | Intragastric Administration (5 mg/kg) | | | |
| | Plasma Clearance (mL/min /kg) | Elimination Half-Life (h) | Apparent Distribution Volume (L/kg) | Area Under the Drug-Time Curve (0-inf, μM·h) | Peak Concentration (μM) | Time to Peak Concentration (h) | Area Under the Drug-Time Curve (0-inf, μM·h) | Bioavailability F (%) |
| Hydrochloride of Compound of Formula (I) | 21.3 | 5.03 | 5.83 | 0.44 | 0.41 | 4.00 | 3.20 | 72.6 |

**Conclusion:** The compound of the present disclosure has high oral systemic plasma exposure ($AUC_{0-inf}$). It has superior pharmacokinetic properties in non-rodent animals such as Beagle dogs.

**Experimental Example 8: *In vivo* pharmacodynamic study of the compound in SCID mouse xenograft tumor model of human B-cell lymphoma OCI-LY10 cells**

**Objective:**

The antitumor effect of the test compound was evaluated using a SCID mouse xenograft tumor model of human B-cell lymphoma OCI-LY10 cells.

**Experimental materials:**

1. Experimental animals: SCID mice, female, 6 to 8 weeks old, weighing 17 to 20 g. Beijing Vital River Laboratory Animal Technology Co., Ltd.
2. Cell line: The human B-cell lymphoma OCI-LY10 cell line was purchased from Nanjing Cobioer Biosciences Co., Ltd., with catalog number CBP60558.

Table 17. Reagent Information

| Name | Manufacturer | Batch Number | Storage Conditions |
|---|---|---|---|
| IMDM Medium | GIBCO, USA | 2323369 | 4°C |
| Fetal Bovine Serum (FBS) | GIBCO, USA | 2305262RP | -20°C |
| Matrigel (Matrigel) | CORNING | 2062001 | -20°C |

Table **18.** Instruments

| Name | Manufacturer | Model |
|---|---|---|
| SHJ Series Clean Bench | Shanghai Shangjing Purification Equipment Co., Ltd. | CA-1390-1 |
| $CO_2$ Water-Jacketed Cell Culture Incubator | Thermo Scientific Forma | 3111 |
| Inverted Microscope | Olympus | CKX41SF |
| Electric Suction Device | Shanghai Medical Instruments (Group) Co., Ltd. | YX930D |
| Eppendorf AG Centrifuge | Eppendorf | 5811XG639987 |
| Balance (0.01 g Precision) | Shanghai Minqiao Precise Science Instrument Co., Ltd. | SL502N |
| Balance (0.001 g Precision) | Shanghai Jinghai Instrument Co., Ltd. | JA2003N |
| Electronic Digital Caliper | SHAHE | (0 to 150) mm |

**Model establishment:**

OCI-LY10 cells were cultured in IMDM medium containing 20% FBS and maintained in a 5% $CO_2$ incubator at 37°C with saturated humidity. OCI-LY10 cells in the logarithmic growth phase were collected, resuspended in IMDM basal medium, mixed 1:1 with Matrigel, and adjusted to a cell concentration of $4 \times 10^7$/mL. Under sterile conditions, 0.1 mL of the cell suspension was subcutaneously inoculated into the right back of SCID mice at an inoculation concentration of $4 \times 10^6$/0.1 mL/mouse.

**Experimental scheme:**

In pharmacodynamic experiments, when the tumors reached a certain size, animals with excessively large or

small tumor volumes or irregular tumor shapes were excluded. Animals with tumor volumes ranging from 167.65 to 231.29 $mm^3$ were selected and randomly grouped based on tumor volume, with 6 mice per group. The average tumor volume was approximately 201.15 $mm^3$. The day of grouping was designated as Day 0, and drug administration was initiated according to the animals' body weights. The pharmacodynamic experiment period was 28 days, with the drug administered once a day with a dosing interval of 24 hours, and administered intragastrically. During the experiment, animal body weight and tumor size were measured twice weekly. Clinical symptoms were observed and recorded daily.

The test compounds were administered at doses of 10 mg/kg, 30 mg/kg, and 100 mg/kg, with the vehicle being 10% DMSO/10% Solutol/80% $H_2O$. Tumor volume (TV) was calculated using the formula: $1/2 \times a \times b^2$, where a and b represent the measured length and width of the tumor, respectively. The tumor growth inhibition rate (TGI, %) was calculated using the formula: TGI (%) = [1 - (average tumor volume at the end of administration in a treatment group - average tumor volume at the beginning of administration in this treatment group) / (average tumor volume at the end of treatment in the vehicle control group - average tumor volume at the beginning of treatment in the vehicle control group)] × 100%. The relative tumor proliferation rate (T/C, %) was calculated using the formula: T/C % = $T_{RTV}$ / $C_{RTV}$ × 100% ($T_{RTV}$: Mean RTV of the treatment group; $C_{RTV}$: Mean RTV of the negative control group). The relative tumor volume (RTV) was calculated based on the tumor measurement results, with the formula RTV = Vt / $V_0$, where $V_0$ is the tumor volume measured at the time of grouping administration *(i.e.,* Day 0), and Vt is the tumor volume at a certain measurement time. $T_{RTV}$ and $C_{RTV}$ were derived from data on the same day.

**Data analysis:**

In this study, experimental data were expressed as Mean ± SEM.

Statistical analysis was performed using IBM SPSS Statistics software based on RTV data at the end of the experiment. Comparisons between two groups were analyzed by T test, while comparisons among three or more groups were analyzed by one-way ANOVA. If the variance was homogeneous (no significant difference in F-value), Tukey's method was applied for analysis. If the variance was heterogeneous (significant difference in F-value), the Games-Howell method was applied for testing. $p < 0.05$ was considered to be significantly different.

**Experimental results:** Test results are shown in Tables **19** and **20.**

Table **19.** Evaluation of the tumor-inhibitory efficacy of the compound of the present disclosure in a subcutaneous xenograft tumor model of human B-cell lymphoma OCI-LY10 cells

| Group | Dosage | Tumor Volume ($mm^3$)[a] (Day 0) | Tumor Volume ($mm^3$)[a] (Day 28) | Relative Tumor Volume[a] (Day 28) | T/C (%) (Day 28) | TGI (%) (Day 28) |
|---|---|---|---|---|---|---|
| Vehicle Control | 0 mg/kg | 201.76 ± 7.08 | 1422.84 ± 89.14 | 7.11 ± 0.56 | -- | -- |
| Hydrochloride of Compound of Formula (I) | 10 mg/kg | 202.43 ± 8.41 | 685.61 ± 75.95 | 3.37 ± 0.34 | 47.40 | 60.43 |
| | 30 mg/kg | 199.80 ± 8.02 | 322.68 ± 20.47 | 1.64 ± 0.17 | 23.07 | 89.94 |
| | 100 mg/kg | 199.75 ± 7.96 | 188.01 ± 35.59 | 0.91 ± 0.15 | 12.80 | 100.96 |
| Note: a. Mean ± SEM. | | | | | | |

Table **20.** *p* values for comparison of relative tumor volumes among groups in the human B-cell lymphoma OCI-LY10 xenograft tumor model treated with the compound of formula (I) of the present disclosure

| Group/Dosage | | Vehicle Control | Hydrochloride of Compound of Formula (I) | | |
|---|---|---|---|---|---|
| | | | 10 mg/kg | 30 mg/kg | 100 mg/kg |
| Vehicle Control | | N/A | 0.003 | 0.001 | <0.001 |
| Hydrochloride of Compound of Formula (I) | 10 mg/kg | 0.003 | N/A | 0.018 | 0.003 |
| | 30 mg/kg | 0.001 | 0.018 | N/A | 0.077 |
| | 100 mg/kg | <0.001 | 0.003 | 0.077 | N/A |
| Note: The *p* value was analyzed using IBM SPSS Statistics software. | | | | | |

**Conclusion:** The compound of the present disclosure exhibits a significant tumor inhibitory effect in the SCID

mouse xenograft tumor model of human B-cell lymphoma OCI-LY10 cells in a dose-dependent manner.

**Experimental Example 9: *In vivo* pharmacodynamic study of the compound in the CB17 SCID mouse subcutaneous xenograft tumor model of human lymphoma SU-DHL-2 cells**

**Objective:**

The antitumor effect of the test compound was evaluated using a SU-DHL-2 subcutaneous xenograft tumor model in CB17 SCID mice.

**Experimental materials:**

1. Experimental animals: CB17 SCID mice, female, 6 to 8 weeks old, weighing 18 to 22 g. Beijing Vital River Laboratory Animal Technology Co., Ltd.
2. Cell line: Human lymphoma SU-DHL-2 cells (Cat. No.: ATCC-CRL-2956).

Table **21.** Reagent Information

| Name | Manufacturer | Cat. No. | Batch Number | Expiration Date | Storage Conditions |
|---|---|---|---|---|---|
| 1640 Medium | Gibco | 22400089 | 2462009 | 2023-02-28 | 4°C |
| Matrigel | Corning | 354234 | 2013002 | 2022-10-19 | -20°C |
| 100× Double Antibiotics (Penicillin, Streptomycin) | Meilunbio | MA0110 | MA0110-Apr-15H | 2023-04-14 | -20°C |
| PBS | HyClone | SH30256.01 | AG29791799 | 2023-08-31 | 4°C |
| Fetal Bovine Serum | ExCell Bio | FND500 | 11H233 | 2023-08 | -20°C |

Table **22.** Instruments

| Name | Manufacturer | Model |
|---|---|---|
| Biosafety Cabinet | Suzhou Antai Airtech Co., Ltd. | BSC-1604IIA2 |
| Vernier Caliper | Mitutoyo | CD-6"ASX |
| Electronic Balance | Changzhou Tianzhiping Instruments Co., Ltd. | EL-2KJ |
| Vortex Vibrator | HaiMen City Qilin Medical Instrument Factory | XW-80A |
| Pure Water System | Millipore | MILLI-Q®Direct8 |

**Model establishment:**

Cell culture: Human lymphoma SU-DHL-2 cells (ATCC-CRL-2956) were cultured in suspension *in vitro* under conditions of RPMI 1640 medium supplemented with 10% inactivated fetal bovine serum, 100 U/mL penicillin, and 100 μg/mL streptomycin, and incubated at 37°C in a 5% $CO_2$ incubator. Routine passaging was performed twice a week. When the cell saturation density was 80% to 90% and the number of cells reached the required level, the cells were collected, counted, and inoculated.

Tumor cell inoculation and grouping: 0.2 mL ($10 \times 10^6$ cells) (PBS: matrigel = 1:1) of SU-DHL-2 cells were subcutaneously inoculated into the right back of each mouse. When the average tumor volume reached approximately 139 $mm^3$, the drugs were administered in groups. The day of grouping was designated as Day 0, and drug administration was initiated according to the animals' body weights.

**Experimental scheme:**

The pharmacodynamic experiment was conducted with a 7-day administration cycle, where the test compound was administered once daily intragastrically at 24-hour intervals, for a total of three cycles. During the experiment, animal body weight and tumor size were measured twice a week, and clinical symptoms were observed and recorded daily.

The test compounds were administered at doses of 10 mg/kg, 30 mg/kg, and 100 mg/kg, with the vehicle being 10% DMSO/10% Solutol/80% water. Tumor volume (TV) was calculated using the formula: $1/2 \times a \times b^2$, where a and b represent the measured length and width of the tumor, respectively. The tumor growth inhibition rate (TGI, %) was calculated using the formula: TGI (%) = [1 - (average tumor volume at the end of administration in a treatment group - average tumor volume at the beginning of administration in this treatment group)) / (average tumor volume at the end of treatment in the vehicle control group - average tumor volume at the beginning of treatment in the vehicle control group)] × 100%. The relative tumor proliferation rate (T/C, %) was calculated using the formula: T/C % = $T_{RTV}$ / $C_{RTV}$ × 100% ($T_{RTV}$: mean RTV of the treatment group; $C_{RTV}$: mean RTV of the negative control group). The relative tumor volume (RTV) was calculated based on the tumor measurement results, with the formula RTV = Vt / $V_0$, where $V_0$ is the tumor volume measured at the time of grouping administration *(i.e.,* Day 0), and Vt is the tumor volume at a certain measurement time. $T_{RTV}$ and $C_{RTV}$ were derived from data on the same day.

**Data analysis:**

Statistical analysis was performed using SPSS software based on RTV data at the end of the experiment. Comparisons between two groups were analyzed by T test, while comparisons among three or more groups were analyzed by one-way ANOVA. If the variance was homogeneous (no significant difference in F-value), Tukey's method was applied for analysis. If the variance was heterogeneous (significant difference in F-value), the Games-Howell method was applied for testing. $p < 0.05$ was considered to be significantly different.

**Experimental results:** Test results are shown in Tables **23** and **24.**

Table **23.** Evaluation of the tumor-inhibitory efficacy of the compound of the present disclosure in the subcutaneous xenograft tumor model of lymphoma SU-DHL-2 cells

| Group/ | Dosage | Tumor Volume ($mm^3$)[a] (Day 0) | Tumor Volume ($mm^3$)[a] (Day 21) | Relative Tumor Volume[a] (Day 21) | T/C (%) (Day 21) | TGI (%) (Day 21) |
|---|---|---|---|---|---|---|
| Vehicle Control | 0 mg/kg | 139 ± 6 | 1883 ± 141 | 13.68 | - | - |
| Hydrochloride of Compound of Formula (I) | 10 mg/kg | 139 ± 6 | 800 ± 48 | 5.79 | 42.33 | 62.13 |
| | 30 mg/kg | 139 ± 6 | 813 ± 99 | 5.78 | 42.26 | 61.41 |
| | 100 mg/kg | 139 ± 5 | 626 ± 51 | 4.55 | 33.23 | 72.07 |
| Note: a. Mean ± SEM. | | | | | | |

Table **24.** *p* values for comparison of relative tumor volumes among groups in the lymphoma SU-DHL-2 xenograft tumor model treated with the compound of the present disclosure

| Group/Dosage | | Vehicle Control | Hydrochloride of Compound of Formula (I) | | |
|---|---|---|---|---|---|
| | | | 10 mg/kg | 30 mg/kg | 100 mg/kg |
| Vehicle Control | | N/A | 0.001 | 0.001 | <0.001 |
| Hydrochloride of Compound of Formula (I) | 10 mg/kg | 0.001 | N/A | 1.000 | 0.268 |
| | 30 mg/kg | 0.001 | 1.000 | N/A | 0.507 |
| | 100 mg/kg | <0.001 | 0.268 | 0.507 | N/A |
| Note: The *p* value was obtained by analyzing the relative tumor volume (RTV) using one-way ANOVA. Data with heterogeneous variance were analyzed using Games-Howell. | | | | | |

**Conclusion:** The compound of the present disclosure exhibits a significant tumor inhibitory effect in a CB17 SCID mouse subcutaneous xenograft tumor model of human lymphoma SU-DHL-2 cells.

**Experimental Example 10: *In vivo* pharmacodynamic study on a BALB/c nude mouse subcutaneous xenograft tumor model of human diffuse large B-cell lymphoma TMD-8 cells**

**Pharmacodynamic study**

**Objective:**

The antitumor effect of the compound of formula (I) was evaluated using a BALB/c nude mouse subcutaneous xenograft tumor model of human diffuse large B-cell lymphoma TMD-8 cells.

**Experimental materials:**

1. Experimental animals: BALB/c nude mice, female, 6 to 8 weeks old. Vital River Laboratory Animal Technology Co., Ltd.
2. Cell line: Human diffuse large B-cell lymphoma TMD-8 cells (purchased from Shanghai Huzhen Industrial Co., Ltd.).

Table **25.** Main Reagent Information

| Name | Manufacturer | Cat. No. | Batch Number |
|---|---|---|---|
| RPMI-1640 Powder | Gibco | 31800-022 | 2383858 |
| Fetal Bovine Serum (FBS) | Gibco | 10099-141C | 2217479CP |
| Matrigel | Corning | 354248 | 2139002 |
| PBS | Beyotime | ST447 | 100921220308 |
| Double Antibiotics | Hyclone | SV30010 | J210026 |
| DMSO | Aladdin | D103273 | H2120209 |
| Solutol HS15 | BASF | 50379938 | 42608009T0 |
| Sterile Water for Injection | Zhejiang Dubang Pharmaceutical | NA | 2111290103 |

Table **26.** Main Instrument Information

| Name | Manufacturer | Model |
|---|---|---|
| Vernier Caliper | Mitutoyo Corporation | CD-6"CX |
| Ultrasonic Homogenizer | Kunshan Ultrasonic Instruments Co., Ltd. | KQ-700DE |
| Balance | METTLER TOLEDO | XP 105 |
| Vortex Mixer | Thermolyne | 16700 |

**Model establishment:**

Cell culture: Routine cell culture was conducted in RPMI-1640 medium containing 10% fetal bovine serum under 5% $CO_2$ at 37°C. Cells were passaged based on cell growth conditions at a passage ratio of 1:3 to 1:4.

Tumor cell inoculation and grouping: TMD-8 cells in the logarithmic growth phase were harvested, counted, and resuspended in a mixture of 50% serum-free RPMI-1640 medium and 50% Matrigel. The cell concentration was adjusted to $4.0 \times 10^7$ cells/mL. The cell suspension was placed in an ice box, aspirated using a 1-mL syringe, and injected subcutaneously into the right forelimb axilla of nude mice at 200 μL per animal ($0.8 \times 10^7$ cells/mouse) to establish the TMD-8 xenograft tumor model. Administration was started when the average tumor volume reached approximately 160 $mm^3$. The day of grouping was designated as Day 1 (D1) of the experiment, and administration was started based on animal body weight.

**Experimental scheme:**

The pharmacodynamic experiment was conducted with a 7-day administration cycle, where the test compound was administered once daily intragastrically at 24-hour intervals, for a total of three cycles. During the experiment, animal body weight and tumor size were measured twice a week, and clinical symptoms were observed and recorded daily.

The test compound was administered at doses of 10 mg/kg, 30 mg/kg, and 100 mg/kg, with the vehicle being 10% DMSO/10% Solutol HS15/80% water. Tumor volume (TV) was calculated using the formula: $1/2 \times a \times b^2$, where a and b represent the measured length and width of the tumor, respectively. The tumor growth inhibition rate (TGI, %) was calculated using the formula: TGI (%) = [1 - (average tumor volume at the end of administration in a treatment group - average tumor volume at the beginning of administration in this treatment group) / (average tumor volume at the end of treatment in the vehicle control group - average tumor volume at the beginning of treatment in the vehicle control group)] × 100%. The relative tumor proliferation rate (T/C, %) was calculated using the formula: T/C % = $T_{RTV} / C_{RTV} \times 100\%$ ($T_{RTV}$: mean RTV of the treatment group; $C_{RTV}$: mean RTV of the negative control group). The relative tumor volume (RTV) was calculated based on the tumor measurement results, with the formula RTV = $V_t / V_0$, where $V_0$ is the tumor volume measured at the time of grouping administration (*i.e.,* Day 1), and Vt is the tumor volume at a certain measurement time. $T_{RTV}$ and $C_{RTV}$ were derived from data on the same day.

**Data analysis:**

The experimental data were calculated and subjected to relevant statistical processing using Microsoft Office Excel 2007 software. Unless otherwise specified, the data were expressed as mean ± standard error (Mean ± SE), and comparisons between groups were performed using t-tests.

**Experimental results:** The test results are shown in Table **27.**

Table **27.** Evaluation of the tumor-inhibitory efficacy of the compound of formula (I) of the present disclosure in the nude mouse xenograft tumor model of human diffuse large B-cell lymphoma TMD-8 cells

| Group | Dosage | Tumor Volume (mm$^3$)$^a$ (Day 0) | Tumor Volume (mm$^3$)$^a$ (Day 22) | Relative Tumor Volume$^a$ (Day 22) | T/C (%) (Day 22) | TGI (%) (Day 22) |
|---|---|---|---|---|---|---|
| Vehicle Control | 0 mg/kg | 160 ± 10 | 1234 ± 179 | 7.62 | - | - |
| Hydrochloride of Compound of Formula (I) | 10 mg/kg | 161 ± 12 | 714 ± 153* | 4.41 | 58 | 49 |
| | 30 mg/kg | 160 ± 9 | 430 ± 98** | 2.60 | 34 | 75 |
| | 100 mg/kg | 160 ± 8 | 291 ± 70** | 1.78 | 23 | 88 |

Note: "*" indicates a significant difference in tumor volume compared to the vehicle control group ($P$ < 0.05); "**" indicates a highly significant difference compared to the vehicle control group ($P$ < 0.01); "*" indicates mean ± SEM.

**Conclusion:** The compound of the present disclosure exhibits a significant tumor inhibitory effect in the BALB/c nude mouse subcutaneous xenograft tumor model of human diffuse large B-cell lymphoma TMD-8 cells.

## Claims

1. A method for preparing compound 6 using compound 4 and compound 5, wherein the reaction step is as follows:

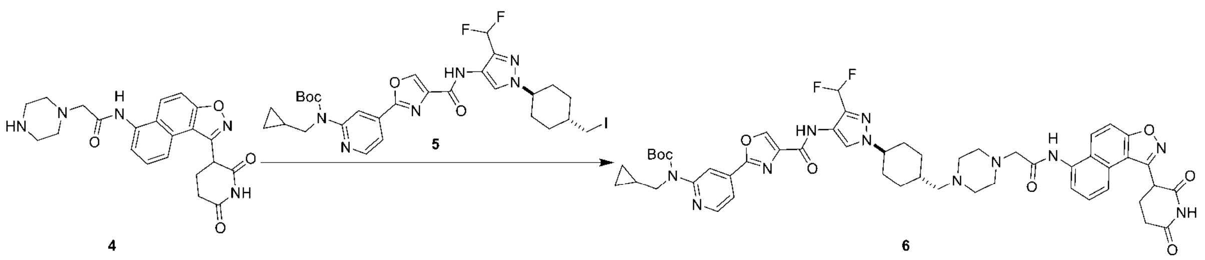

4 5 6

.

2. A method for preparing a compound of formula (I) using compound 1, wherein the reaction steps are as follows:

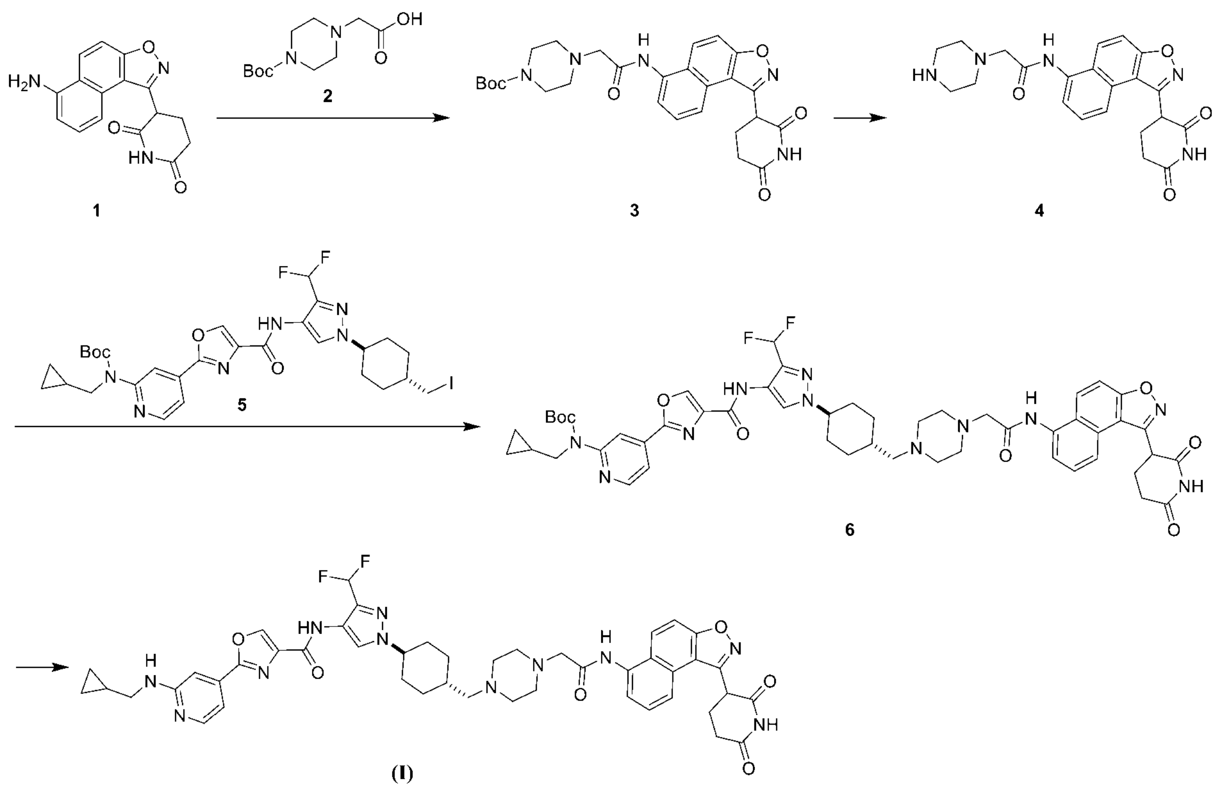

1 2 3 4 5 6 (I)

.

3. The method according to claim 1, wherein

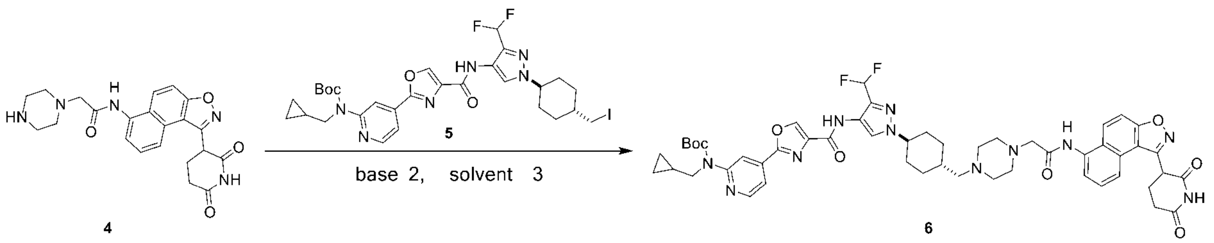


4 5 6

the equivalent ratio of compound 4 to compound 5 is 0.8:1 to 1:1;
base 2 is selected from the group consisting of TEA and DIEA;
solvent 3 is acetonitrile.

4. The method according to claim 3, wherein the reaction temperature is 70 to 80°C.

5. The method according to claim 3, wherein the equivalent ratio of compound 4 to compound 5 is 0.85:1 to 0.95:1; preferably 0.88:1.

6. The method according to claim 2, wherein

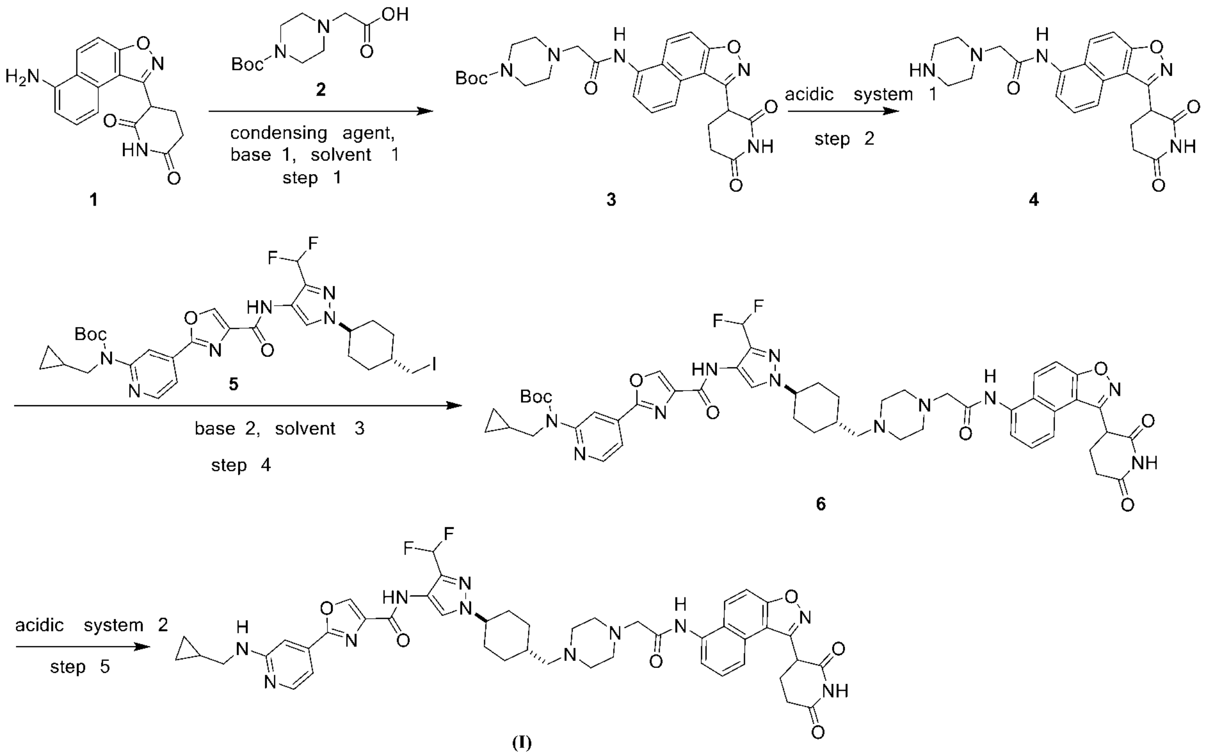


1 2 3 4 5 6 (I)

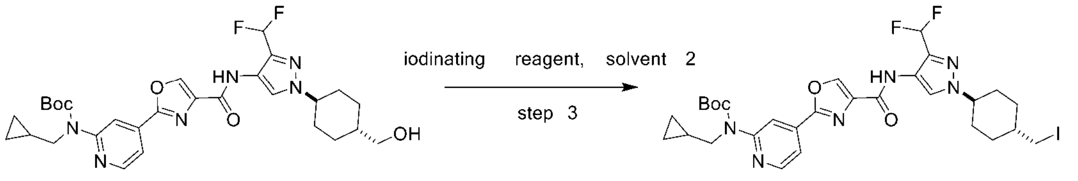


5-1 5

,

the condensing agent is selected from the group consisting of HATU, benzenesulfonyl chloride, and *p*-toluenesulfonyl chloride;
base 1 is selected from the group consisting of TEA and DIEA;
solvent 1 is selected from the group consisting of DMF, DMSO, and NMP;
acidic system 1 is selected from the group consisting of a solution of hydrogen chloride in ethyl acetate and TFA;
acidic system 2 is selected from the group consisting of a solution of hydrogen chloride in ethyl acetate and TFA;
base 2 is selected from the group consisting of TEA, DIEA, and sodium bicarbonate;
solvent 3 is acetonitrile;
the iodinating reagent is $PPh_3/Im/I_2$;
solvent 2 is DCM.

**7.** The method according to claim 6, wherein the condensing agent is HATU; acidic system 1 is TFA; acidic system 2 is a solution of hydrogen chloride in ethyl acetate, wherein the concentration of the solution of hydrogen chloride in ethyl acetate is 4 mol/L; base 1 is DIEA; base 2 is DIEA.

**8.** The method according to claim 6, wherein the reaction temperature of step 5 is 40 to 50°C.

**9.** The method according to claim 8, wherein the reaction time of step 5 is 0.5 to 3 hours.

## INTERNATIONAL SEARCH REPORT

International application No.

**PCT/CN2024/083383**

**A. CLASSIFICATION OF SUBJECT MATTER**

C07D 413/14(2006.01)i; C07D498/00(2006.01)i

According to International Patent Classification (IPC) or to both national classification and IPC

**B. FIELDS SEARCHED**

Minimum documentation searched (classification system followed by classification symbols)

IPC: C07D413/-: C07D498/-

Documentation searched other than minimum documentation to the extent that such documents are included in the fields searched

Electronic data base consulted during the international search (name of data base and, where practicable, search terms used)

CNABS; CNTXT; VEN; WOTXT; USTXT; EPTXT; GBTXT; CATXT; EPTXT; CNKI; 万方, WANFANG; STN: 萘并异噁唑, 哌啶二酮, 戊二酰亚胺, 哌嗪, 碘, 二异丙基乙胺, 三乙胺, 蛋白降解靶向嵌合体, 林锦霞, 沈国婴, 杨丽美, 吴骅楷, 张晓琴, 王绍辉, 邢少廷, 徐雨, 漳州片仔癀药业股份有限公司, 南京明德新药研发有限公司, +naphtho+, +oxazol+, +piperidinedione+, +glutarimide+, +piperazine+, iodine, I, iodinat+, DIEA, TEA, Proteolysis Targeting Chimera, PROTAC, CAS号2918284-14-5, CAS号2919995-09-6, CAS号2919995-11-0, CAS号2919995-11-0, 权利要求1和权利要求2中的反应式

**C. DOCUMENTS CONSIDERED TO BE RELEVANT**

| Category* | Citation of document, with indication, where appropriate, of the relevant passages | Relevant to claim No. |
|---|---|---|
| PX | WO 2023045978 A1 (MEDSHINE DISCOVERY INC.) 30 March 2023 (2023-03-30) description, page 33, pages 80-82, embodiment 14, and pages 95-106, embodiments 23-29 | 1-9 |
| A | CN 115772180 A (MEDSHINE DISCOVERY INC.) 10 March 2023 (2023-03-10) paragraph, paragraphs [0584], [0601]-[0603], and [0642]-[0693] | 1-9 |
| A | WO 2022262782 A1 (MEDSHINE DISCOVERY INC.) 22 December 2022 (2022-12-22) entire document | 1-9 |
| A | WO 2020048548 A1 (CHIA TAI TIANQING PHARMACEUTICAL GROUP CO., LTD. et al.) 12 March 2020 (2020-03-12) entire document | 1-9 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **2024年05月21日** | **30 May 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2024/083383**

| Patent document cited in search report | | | Publication date (day/month/year) | Patent family member(s) | | | Publication date (day/month/year) |
|---|---|---|---|---|---|---|---|
| WO | 2023045978 | A1 | 30 March 2023 | None | | | |
| CN | 115772180 | A | 10 March 2023 | TW | 202317097 | A | 01 May 2023 |
| | | | | WO | 2023036175 | A1 | 16 March 2023 |
| WO | 2022262782 | A1 | 22 December 2022 | None | | | |
| WO | 2020048548 | A1 | 12 March 2020 | CA | 3111649 | A1 | 12 March 2020 |
| | | | | AU | 2019334065 | A1 | 15 April 2021 |
| | | | | IL | 281296 | A | 29 April 2021 |
| | | | | IL | 281296 | B1 | 01 April 2024 |
| | | | | EP | 3848367 | A1 | 14 July 2021 |
| | | | | EP | 3848367 | A4 | 08 June 2022 |
| | | | | BR | 112021004269 | A2 | 25 May 2021 |
| | | | | US | 2022119376 | A1 | 21 April 2022 |
| | | | | KR | 20210057767 | A | 21 May 2021 |
| | | | | SG | 11202102271 | WA | 29 April 2021 |
| | | | | JP | 2021536480 | A | 27 December 2021 |
| | | | | JP | 7422139 | B2 | 25 January 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2023102949123